# EUROPEAN PATENT APPLICATION

(11) **EP 1 312 325 A2**
(43) Date of publication of application: **21.05.2003**
(21) Application number: 02257679.7
(22) Date of filing: 06.11.2002
(51) Int. Cl.: A61F 13/15

(54) **Disposable diaper**

(30) Priority: 07.11.2001 JP 2001342552
(71) Applicant: Uni-Charm Corporation, Kawanoe-shi, Ehime-ken 182 (JP)
(72) Inventor: Ishikawa, Hiroki, c/o Technical Center, Mitoyo-gun, Kagawa-ken 769-1602 (JP); Kinoshita, Akiyoshi, c/o Technical Center, Mitoyo-gun, Kagawa-ken 769-1602 (JP)
(74) Representative: Parry, Christopher Stephen

(57) **Abstract**

A disposable diaper 1 has an absorbent core 4 comprising hydrophilic fibers 26 and super-absorbent polymer particles 27. The core 4 contains therein the hydrophilic fibers 26 of 30 - 70 wt% and the super-absorbent polymer particles 27 of 70 - 30 wt%. The super-absorbent polymer particles 27 have a liquid-absorbing duration of 3 - 30 seconds and a liquid-permeating duration of 15 - 100 seconds.

## Description

This invention relates to disposable diapers for absorption and containment of excrement.

Japanese Patent Publication No. 1977-20692A disclosed several embodiments of a body fluid absorbent core used in a sanitary napkin, each comprising fluff pulp fibers and super-absorbent polymer particles. The embodiments include the core comprising the super-absorbent polymer particles and the fluff pulp fibers homogeneously mixed together and the core comprising a layer of aggregated super-absorbent polymer particles and two layers of fluff pulp fibers sandwiching the layer of polymer particles from above and below, respectively.

It is well known that a water holding capacity of the super-absorbent polymer particles is substantially higher than a water holding capacity of fluff pulp fibers and use of the super-absorbent polymer particles is effective to prevent a so-called rewet phenomenon. However, once having formed a gel block, the super-absorbent polymer particles may have an effect opposite to what was expected for the polymer particles. To avoid this, it is preferred for the core comprising a mixture of the super-absorbent polymer particles and the fluff pulp fibers to mix them together as homogeneously as possible and thereby to prevent the individual super-absorbent polymer particles from coming in contact one with another. To this end, however, a problem is faced that a content of the super-absorbent polymer particles in the core should be inevitably limited. As one of measures to solve this problem, it is well known to use a mixture of the super-absorbent polymer particles having a relatively high absorption rate and the super-absorbent polymer particles having a relatively low absorption rate. In this case, two different types of super-absorbent polymer particles must be mixed with the fluff pulp fibers as homogeneously as possible. Here arises another problem that a significant difference in specific gravity between the super-absorbent polymer particles and the fluff pulp fibers makes it difficult to mix them to a desired homogeneity. The sandwich type core including the layer of aggregated super-absorbent polymer particles, on the other hand, is accompanied with a problem that the super-absorbent polymer particles readily form the gel block as the polymer particles absorb body fluids and a certain amount of body fluids stagnates above the gel block. The amount of body fluids stagnating above the gel block may flow back toward a sanitary napkin or a diaper wearer's skin as body weight of the wearer is exerted upon such core. In consequence, a remarkable rewet phenomenon may occur and give the wearer an uncomfortable feeling of wetness.

In view of the problems as have been described above, it is an object of this invention to provide a disposable diaper in which the body fluid absorbent core containing therein the super-absorbent polymer particles has an improved preventive effect against the rewet phenomenon.

According to this invention, there is provided a disposable diaper having a body facing surface and a garment facing surface wherein a liquid-pervious topsheet defining the body facing surface is provided on an inner side thereof with an absorbent core.

The core comprises hydrophilic fibers of 30 - 70 wt% and super-absorbent polymer particles of 70 - 30 wt% wherein the super-absorbent polymer particles have a liquid-absorbing duration of 3 - 30 seconds and a liquid-permeating duration of 15 - 100 seconds.

This invention includes the following embodiments.

The diaper has an instantaneous liquid-uptake in a range of 0.5 - 3.0 g per 1 cm² of the surface of the core opposite to the topsheet.

The core comprises a substantially homogeneous mixture phase of the hydrophilic fibers and the super-absorbent polymer particles.

The disposable diaper has a front waist region, a rear waist region and a crotch region extending between the waist regions. The core extends over the crotch region into the front and rear waist regions and, in the front and rear waist regions as well as in the crotch region or at least in the crotch region. The core has a polymer phase primarily comprising the super-absorbent polymer particles and extending in a middle zone as viewed in a transverse direction as well as in a thickness direction of the core and a mixture phase comprising substantially homogeneous mixture of the hydrophilic fibers and the super-absorbent polymer particles and extending above and below as well as outside the polymer phase.

The polymer phase has, in the crotch region, a width corresponding to 40 - 90 % of a full width of the core.

The core has a longitudinal direction orthogonal to the transverse direction and the polymer phase has a length corresponding to 40 - 100 % of a dimension of the core as measured in the longitudinal direction.

The surface of the core opposite to the topsheet has an area of 750 - 1500 cm².

The diaper has an instantaneous liquid-uptake in a range of 1.1 - 2.5 g per 1 cm² of the surface of the core opposite to the topsheet.

The parameter "liquid-absorbing duration" of the super-absorbent polymer particles herein described is measured using a method as follows. First, 50 g of physiologic saline poured into a 100 ml beaker is stirred at a temperature of 25°C by a magnetic stirrer with a rotor sized in 8 mm diameter X 30 mm length and having a revolution speed of 600 rpm. Then 2.0 g of the super-absorbent polymer particles is put into the beaker and an elapsed time is measured before revolving vortex of the physiologic saline disappears and the saline level becomes flat. The time elapse measured in this manner is obtained as "liquid-absorbing duration".

The parameter "liquid-permeating duration" of the super-absorbent polymer particles herein described is measured using a method as follows. First, a nylon filter of 250 meshes is attached to a bottom side of a cylinder having a diameter of 39 mm and 1 g of the super-absorbent polymer particles is evenly spread. Then, 10 seconds are spent to pour 50 g of the physiologic saline into the cylinder. It is assumed that the super-absorbent polymer particles are saturated after such a step of pouring has been repeated six times at time intervals of 3 minutes. 3 minutes after the sixth step of pouring, 10 seconds are spent to pour 50 g of the physiologic saline into the cylinder and a time period elapsing until substantially total amount of this physiologic saline permeates the filter is measured. The time elapse measured in this manner is obtained as "liquid-permeating duration".

The parameter "instantaneous liquid-uptake" of the disposable diaper herein described is measured using a method as follows. Of the disposable diaper using the core of which the "instantaneous liquid-uptake is to be measured, the elastic members contracted inwaist- and leg-surrounding directions are cut off at one or more positions thereof and, in the case of the pants-type diaper, transversely opposite side edge portions thereof also are decoupled back and forth to develop the diaper in flat state. After a weight W₁ of the diaper has been measured, the diaper with the topsheet facing downward is immersed into 0.9 % physiologic saline (20°C) for 5 seconds and then taken out from the saline. The diaper is then folded with the topsheet outside to place the front and rear waist regions upon each other and hung for 1 minute with the crotch region lying on its bottom side. 1 minute after, a weight W₂ of the diaper is measured and a difference between the weights W₁ and W₂ is divided by the area of the core surface opposite to the topsheet. The value calculated in this manner is obtained as "instantaneous liquid-uptake" per 1 cm² of the core's surface area in the disposable diaper.
Fig. 1 is a plan view of a partially cutaway disposable diaper according to this invention;
Fig. 2 is a cross-sectional view taken along a line II - II in Fig. 1;
Fig. 3 is a cross-sectional view taken along a line III - III in Fig. 1;
Fig. 4 is a view similar to Fig. 2 but showing an alternative embodiment of this invention; and
Fig. 5 is a view similar to Fig. 3 but showing this alternative embodiment of this invention.

Details of a disposable diaper according to this invention will be more fully understood from the description given hereunder with reference to the accompanying drawings.

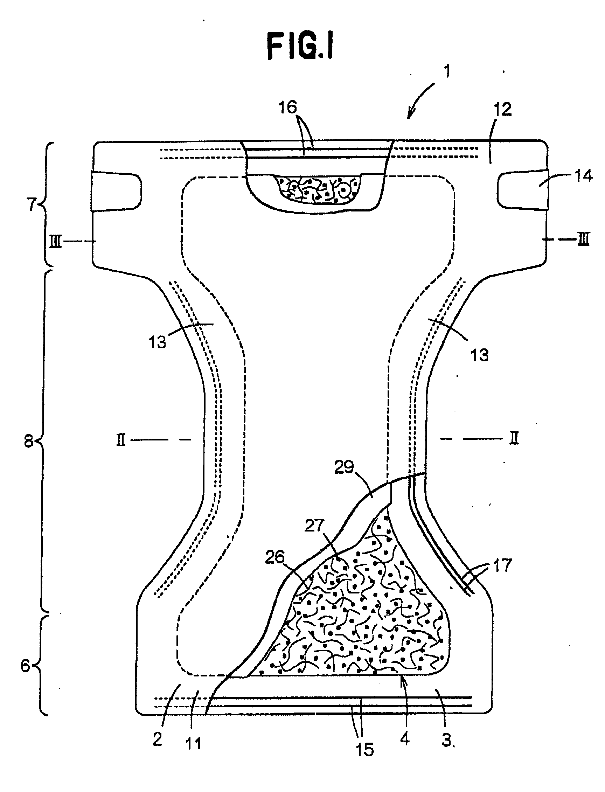

Fig. 1 is a plan view of a partially cutaway disposable diaper 1 according to this invention. This diaper 1 is of open-type and comprises a liquid-pervious topsheet 2 defining a body facing surface, a liquid-impervious backsheet 3 defining a garment facing surface and an absorbent core 4 interposed between the two sheets 2, 3. Portions of the top- and backsheets 2, 3 extending outward beyond a peripheral edge of the core 4 are placed upon and joined to each other to form front and rear end flaps 11, 12 and a pair of side flaps 13. The diaper 1 is composed, in its longitudinal direction (i.e., vertical direction as viewed in Fig. 1), a front waist region 6, a rear waist region 7 and a crotch region 8 extending between the two waist-regions 6, 7. In the rear waist region 7, the pair of side flaps 13 are provided with tape fasteners 14 attached thereto, respectively. In the front and rear end flaps 11, 12 and the side flaps 13 in the crotch region 8, the waist-surrounding elastic members 15, 16 and the leg-surrounding elastic members 17 are secured in a stretched state to the inner surface of at least one of the top- and backsheets 2, 3.

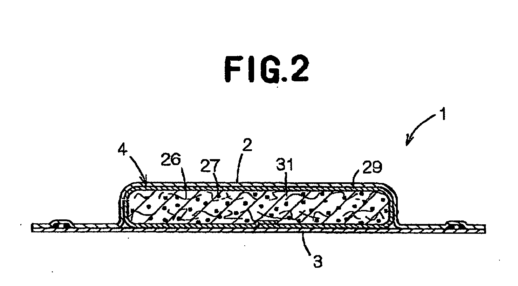

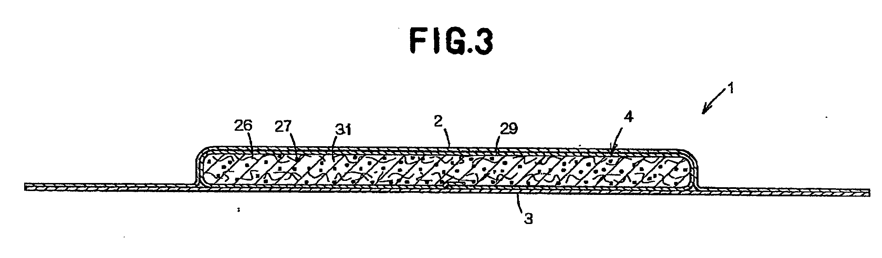

Fig. 2 is a cross-sectional view taken along a line II - II in Fig. 1 and Fig. 3 is a cross-sectional view taken along a line III - III in Fig. 1. In the diaper 1, a stock material for the topsheet 2 may be a nonwoven fabric or a porous film. The nonwoven fabric used for this diaper 1 may be selected from a group consisting of thermoplastic synthetic fibers such as polyolefine-, polyester- or polyamide-based thermoplastic synthetic fibers, and polyethylene/polypropylene or polyethylene/polyester conjugated fibers of sheath-and-core type or side-by-side type. It is also possible to use nonwoven fabric of hydrophilically modified these fibers or nonwoven fabric of water-absorbent fibers such as rayon, acetate and cotton. The porous film may be, for example, a polyethylene film.

A stock material for the backsheet 3 may be selected from a group including polyethylene-, polypropylene-, polyester- and polyurethane films, which are preferably of breathable type.

By reference to Figs. 2 and 3 in conjunction with Fig.1, it will be apparent that the core 4 is hourglass-shaped with a relatively large width in the front and rear waist regions 6, 7 and a relatively small width in the crotch region 8. The core 4 comprises hydrophilic fibers 26 such as fluff pulp fibers and super-absorbent polymer particles 27. The hydrophilic fibers 26 and polymer particles 27 are mixed together as homogeneously as possible to form a mixture phase 31 which is, in turn, covered with a tissue paper or a high wet strength nonwoven fabric made of hydrophilically modified thermoplastic synthetic fibers. The core 4 preferably has a thickness in a range of 3 - 15 mm and an area of its surface opposite to the topsheet 2 in a range of 300 - 1800 cm².

In the core 4, the mixture phase 31 comprising the hydrophilic fibers of 20 - 90 wt%, preferably of 30 - 70 wt% and the polymer particles 27 of 80 - 10 wt%, preferably of 70 - 30 wt% is compressed to ensure its density preferably in a range of 0.06 - 0.15 g/cm³. The hydrophilic fibers 26 may be selected from a group consisting of natural fibers such as fluff pulp fibers, cotton or jute, regenerated fibers such as rayon or acetate and hydrophilically modified thermoplastic synthetic fibers. The polymer particles 27 have a liquid-absorbing duration of 3 - 30 seconds and a liquid-permeating duration of 15 - 100 seconds. At least 50 wt% of the polymer particles 27 have a particle diameter of 250 - 600 µm, preferably of 300 - 400 µm.

In the diaper 1 having the core 4 constructed in this manner, urine discharged on the diaper 1 is absorbed and diffused by the tissue paper 29 lying on the upper surface of the core 4. Simultaneously, urine is absorbed by the hydrophilic fibers 26 and moves down in the thickness direction of the core 4. During downward movement, urine is absorbed also by the polymer particles 27 having a short liquid-absorbing duration and a high liquid-absorbing rate. The liquid-permeating duration of the polymer particles 27 measured with the particles 27 closely aggregated is relatively short. If these particles 27 are mixed with the hydrophilic fibers 26 not in so closely aggregated condition and even a large amount of urine discharged at once or repeatedly discharged several times can easily permeate a layer of the polymer particles and move toward the bottom of the core without stagnating in the vicinity of the upper surface of the core 4, it is possible to avoid a so-called rewet phenomenon that a certain amount of urine flows back as the wearer's body weight is exerted upon the core 4. Such diaper 1 preferably has the instantaneous liquid-uptake of 0.5 - 3.0 g per 1 cm² of the area of the core's surface opposite to the topsheet 2. In the case of the diaper 1 having the instantaneous liquid-uptake less than 0.5 g, urine leakage would be inevitable if a urination flow rate is relatively high depending on the particular wearer. The diaper 1 having the instantaneous liquid-uptake exceeding 3 g, on the other hand, would necessarily contain an unacceptably excessive amount of both the hydrophilic fibers 26 and the polymer particles 27 and become bulky, so a feeling to wear the diaper 1 would be deteriorated. For the wearer who discharges a relatively large amount of urine, for example, 300 g or more for every urination or for the wearer whose urination flow rate is as high as 20 ml/sec or more, the surface area of the core 4 is preferably in a range of 750 - 1500 cm² and the instantaneous liquid-uptake is preferably in a range of 1.1 - 2.5 g. An example of such particular wearers is adult female. While the surface area of the core 4 is limited, in the case of the diaper 1 of pants-type, the core 4 having the surface area and the instantaneous liquid-uptake in the above-mentioned ranges is particularly effective for prevention of urine leakage.

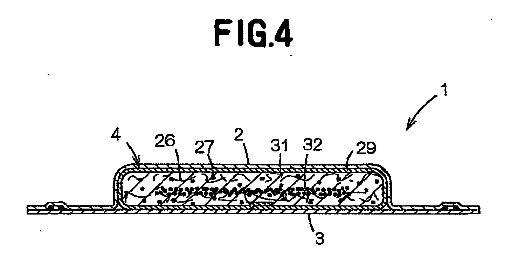

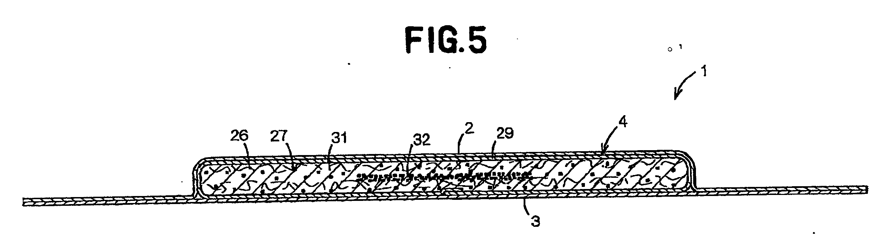

Fig. 4 is a view similar to Fig. 2 but showing an alternative embodiment of this invention and Fig. 5 is a view similar to Fig. 3 but showing this alternative embodiment of this invention. The core 4 comprises the hydrophilic fibers 26 and the super-absorbent polymer particles 27. More specifically, the core 4 has, in addition to the mixture phase 31 formed by a substantially homogeneous mixture of the hydrophilic fibers 26 and the polymer particles 27, a polymer phase 32 formed primarily by the polymer particles 27 and the two phases 31, 32 are covered with the tissue paper 29. This core 4 also preferably has a thickness of 3 - 15 mm.

The mixture phase 31 comprises the hydrophilic fibers 26 of 30 - 95 wt% and the polymer particles 27 of 70 - 5 wt%. The mixture phase 31 is compressed so as to have a density of 0.06 - 0.15 g/cm³.

The polymer phase 32 contains the polymer particles 27 of 90 wt% or higher and lies, in the front and rear waist regions and crotch region or at least in the crotch region, in a middle zone of the core 4 as viewed in a transverse direction as well as in a thickness direction of the core 4. In other words, the mixture phase 31 lies in the core 4 above and below as well as laterally outside the polymer phase 32. In such crotch region 8, the preferable polymer phase 32 has a width corresponding to 40 - 90 % of the full width of the core 4 but, in the front and rear waist regions 6, 7, may have a width corresponding to less than 40 % of the full width of the core 4. The polymer phase 32 has a length corresponding to 40 - 100 % of the full length of the core 4. The polymer phase 32 contains the polymer particles 27 at a ratio of 50 - 600 g/m² and is compressed so as to have a thickness of 0.3 - 3.5 mm and a density of 0.09 - 0.47 g/cm³.

In the diaper 1 having the core 4 constructed in this manner, urine discharged on the diaper 1 is absorbed and diffused by the tissue paper 29 lying on the upper surface of the core 4. Simultaneously, urine is absorbed by the hydrophilic fibers 26 and moves down in the thickness direction of the core 4. In the transversely middle zone of the core 4, urine is absorbed also by the polymer particles 27 as urine reaches the polymer phase 32. In the transversely opposite side zones of the core 4, the mixture phase 31 is continuous in the thickness direction of the core 4 and allows discharged urine to move down to the bottom of the core 4. In the transversely middle zone of the core 4, a part of urine moving downward moves also to a zone of the mixture phase 31 underlying the polymer phase 32. The liquid-permeating duration of the polymer particles 27 forming the polymer phase 32 is relatively short and therefore, even if a large amount of urine is discharged at once or urination is repeatedly several times, urine is absorbed by the polymer particles 27 in the course of being absorbed by the hydrophilic fibers 26 and moving downward.

Such a manner in which discharged urine is absorbed by the core 4, more specifically, absorbed not only by the polymer particles 27 in the mixture phase 31 and the polymer particles 27 in the polymer phase 32 but also by the hydrophilic fibers 26 and the polymer particles 27 both underlying the polymer phase 32 is effective to prevent a so-called rewet phenomenon possibly occurring as the body weight of the diaper wearer is exerted upon the core 4. While the polymer particles 27 in the polymer phase 32 may often form the gel block, such gel block serves to prevent the amount of urine having been absorbed by the portion of the mixture phase 31 underlying the polymer phase 32 from flowing back toward the diaper wearer's skin.

This invention is applicable not only to the open-type diaper as illustrated as the specific embodiments but also to the pants-type diaper.

### (EXAMPLE)

A diaper having the planar shape as shown in Fig. 1 was made using super-absorbent polymer particles A, B. Top surface of a core had an area of 940 cm² and the core itself was contoured as seen in Figs. 2 and 3. Manufacturing conditions and the measured amount of rewet for the core in the diaper according to this invention are indicated in columns of EXAMPLES 1, 2 and 3 of TABLE 1. EXAMPLE 1 is the case in which the core has only the mixture phase comprising the super-absorbent polymer particles and the fluff pulp and EXAMPLES 2 and 3 are the cases in which the core has, in addition to the mixture phase, the polymer phase. The amount of rewet indicated in TABLE 1 was measured in the manner as follows. The diaper was flatly developed and a cylinder having an inner diameter of 60 mm and a weight of 750 g was placed on the middle zone of the core. From 10 mm above the diaper, 150 ml of physiologic saline was poured into the cylinder at a flow rate of 10 ml/sec. After the physiologic saline had been absorbed by the core, the cylinder was removed. 5 minutes after the start of pouring, a 100 X 100 mm paper filter had previously been checkweighed was placed on the position of the core at which the cylinder had been placed, then a weight of 3.5 kg/100 X 100 mm was placed on the paper filter and left as it is for 3 minutes. Thereafter the paper filter was checkweighed to determine a liquid-uptake during this time period of 3 minutes as the amount of rewet (of first step). 10 minutes after the start of pouring the physiologic saline, the cylinder was placed again on the central region of the core and 150 ml of physiologic saline was poured into the cylinder to measure the liquid-uptake by the paper filter as the amount of rewet (of second step). The similar procedure was repeated once more to obtain the amount of rewet (of third step).

### (CONTROL)

In the case of the diaper as a control, super-absorbent polymer particles C were used for the core. The polymer particles C having a liquid-absorbing duration of 3 seconds and a liquid-permeating duration of 180 seconds were homogeneously mixed with fluff pulp fibers to form the mixture phase. Manufacturing conditions and the amount of rewet of this diaper is indicated in the column of CONTROL of TABLE 1.

As will be apparent from TABLE 1, the core using the super-absorbent polymer particles characterized by relatively short liquid-absorbing duration and liquid-permeating duration exhibited the amount of rewet smaller than that of the super-absorbent polymer particles having the liquid-permeating duration exceeding 180 seconds. For example, the former exhibited the amount of rewet as small as less than 75 g even in third step and the preventive effect against back flow of body fluids exhibited by the former is substantially higher than that exhibited by the core as CONTROL.

**[TABLE 1]**

| | Examples | | | Control |
|---|---|---|---|---|
| | 1 | 2 | 3 | |
| Polymer particles | | | | |
| types | A | A | B | C |
| liquid-absorbing duration (sec) | 14 | 14 | 25 | 3 |
| liquid-permeating duration (sec) | 71 | 71 | 30 | 180 |
| | | | | |
| Mixture phase (g/m²) | 680 | 564 | 564 | 680 |
| fluff pulp fibers(g/m²) | 426 | 426 | 426 | 425 |
| polymer particles (g/m²) | 254 | 138 | 138 | 255 |
| | | | | |
| Polymer phase (g/m²) | ― | 229 | 229 | ― |
| | | | | |
| Total thickness (mm) | 7.5 | 7.5 | 7.5 | 7.5 |
| | | | | |
| Thickness of polymer phase | ― | 1.5 | 1.5 | ― |
| Area of polymer phase (cm²) | ― | 480 | 480 | ― |
| Amount of rewet | | | | |
| first step (g) | 1.2 | 1.1 | 0.2 | 5.6 |
| second step (g) | 55 | 49 | 31 | 67 |
| third step (g) | 73 | 69 | 58 | 87 |
| Area of core surface opposite | | | | |
| to surface sheet (cm²) | 940 | 940 | 940 | 940 |
| Instantaneous liquid-uptake | 1.27 | 1.28 | 0.83 | 1.36 |

The disposable diaper according to this invention is based on the improvement that the absorbent core includes the homogeneous mixture phase comprising the super-absorbent polymer particles having the liquid absorbing duration as well as the liquid permeating duration both of which are relatively short and the hydrophilic fibers or, in addition to such mixture phase, the polymer phase primarily comprising the super-absorbent polymer particles. With such improvement, not only these polymer particles reliably absorb the body fluids to avoid a so-called rewet phenomenon but also the polymer phase prevents the amount of the body fluids once retained under the polymer phase from moving upward and thereby to avoid the rewet phenomenon more reliably.

## Claims

1. A disposable diaper having a body facing surface and a garment facing surface wherein a liquid-pervious topsheet defining the body facing surface is provided on an inner side thereof with an absorbent core, said disposable diaper further comprising:
said core comprising hydrophilic fibers of 30 - 70 wt% and super-absorbent polymer particles of 70 - 30 wt% wherein said super-absorbent polymer particles have a liquid-absorbing duration of 3 - 30 seconds and a liquid-permeating duration of 15 - 100 seconds.

2. The disposable diaper according to Claim 1, wherein said diaper has an instantaneous liquid-uptake in a range of 0.5 - 3.0 g per 1 cm² of the surface of said core opposite to said topsheet.

3. The disposable diaper according to Claim 1, wherein said core comprises a substantially homogeneous mixture phase of said hydrophilic fibers and said super-absorbent polymer particles.

4. The disposable diaper according to Claim 1, wherein said disposable diaper has a front waist region, a rear waist region and a crotch region extending between these waist regions and wherein said core extends over said crotch region into said front and rear waist regions and, in said front and rear waist regions and crotch region or at least in said crotch region, said core has a polymer phase primarily comprising said super-absorbent polymer particles and lying in a middle zone as viewed in a transverse direction as well as in a thickness direction of said core and a mixture phase comprising substantially homogeneous mixture of said hydrophilic fibers and said super-absorbent polymer particles and lying above and below as well as laterally outside said polymer phase.

5. The disposable diaper according to Claim 4, wherein said polymer phase has, in said crotch region, a width corresponding to 40 - 90 % of a full width of said core.

6. The disposable diaper according to Claim 4, wherein said core has a longitudinal direction orthogonal to said transverse direction and said polymer phase has a length corresponding to 40 - 100 % of a dimension of said core as measured in said longitudinal direction.

7. The disposable diaper according to Claim 1, wherein the surface of said core opposite to said topsheet has an area of 750 - 1500 cm².

8. The disposable diaper according to Claim 7, wherein said diaper has an instantaneous liquid-uptake in a range of 1.1 - 2.5 g per 1 cm² of the surface of said core opposite to said topsheet.
